# EUROPEAN PATENT APPLICATION

(11) **EP 3 766 468 A1**
(43) Date of publication of application: **20.01.2021**
(21) Application number: 19187424.7
(22) Date of filing: 19.07.2019
(51) Int. Cl.: A61F 13/56, A61F 13/62

(54) **CLOSURE TAPE TAB WITH AN INNER TAB PORTION HAVING MECHANICAL FASTENING ELEMENTS**

(71) Applicant: 3M Innovative Properties Company, Saint Paul, MN 55133-3427 (US)
(72) Inventor: Genç, Mr. Yavuz, Corlu/Tekirdag (TR); Nebigil, Mr. Can, Tekirdag (TR); Yurdakurban, Mr. Do an, 59500 Cerkezkoy, Tekirdag (TR); Abakay, Ms. Ayca, 59500 Cerkezkoy, Tekirdag (TR)
(74) Representative: Vollmers, Hans-Gerd

(57) **Abstract**

The present disclosure relates to a closure tape tab 10 with a proximal and a distal end portions 20, 30 connected by an inner portion 40 from which mechanical fastening elements 48 protrude. The present disclosure further relates to such a closure tape 100, optionally wound into a roll 110, and to a method of making such a closure tape 100 and such closure tape tabs 10. The present disclosure moreover relates to an absorbent article 115 such as a disposable diaper 115 having such closure tape tabs 10.

## Description

The present disclosure relates to a closure tape tab for fastening of an absorbent article with a proximal and a distal end portion connected by an inner tab portion from which mechanical fastening elements protrude. The present disclosure further relates to such a closure tape, optionally wound into a roll, and to a method of making such a closure tape and such closure tape tabs. The present disclosure moreover relates to a disposable absorbent article having such closure tape tabs.

A disposable diaper typically has a thin, flexible, stretchy, low density polyethylene back sheet film, an absorbable core on the inside of the backsheet film and a porous top sheet overlying the core. The diaper is positioned at the crotch of the wearer, two ends of the diaper extending, respectively, towards the front and back. A wearer is understood to be the same person as a user of an absorbent article or a different person giving care to the wearer of the absorbent article. The terms wearer and user are thus used with a similar meaning throughout this specification. Adjacent edges of the diaper at each side are then either positioned adjacent to each other or overlapped. A closure tape tab is used to close the diaper. Typically, the proximal end portion is oriented towards the diaper. The closure tape tab is typically attached to the diaper with its proximal end portion, for example by means of an adhesive layer. The opposite end portion (distal end portion) of the closure tape tab typically comprises a fastening means (e. g., a pressure sensitive adhesive layer or a mechanical fastener component) to close the diaper around the wearer's body and fasten it on the body. Closure tape tabs with a proximal and a distal end portion are described, for example, in EP 1 133 967 A1 (3M, Kinnear et al.) or EP 1 697 134 B1 (3M, Selen et al.).

Known closure tape tabs used in, for example, disposable articles such as disposable diapers, sanitary napkins, pant-style diapers etc. comprise a support layer or backing bearing on one of its major surfaces a patch of (mechanical) fastening means in order to securely join parts of the article together. Said closure tape tab may, for example, comprise a hook patch which usually extends across the whole width of the closure tape tab. Therefore, the width and length of the hook patch is adding to the bending performance of the closure tape tab. Since the hook patch comprises a rather rigid material, this may cause the problem that the closure tape tab may be not flexible enough to conform to a movement of the wearer and thus may decrease the comfort of wearing as well as may provide the risk of unintentional pop-open of the closure tape tab with a limited bendability.

Attempts were made to reduce the rigidity of the hook patch. For example, slits or incisions are made to the support layer of the hook patch in order to reduce the rigidity, see for example EP 1 838 178 A1 (3M, Oertel). Other attempts were made such as dividing the hook patch into several smaller hook patches or strips, see for example EP 1 635 752 A1 (3M, Petersen et al.). However, these solutions require additional process steps such as applying cuts in the hook patch. Also, the support layer of the closure tape tab needs to extend continuously underneath of the hook patch.

Therefore, a need exists to provide a closure tape tab with an improved flexibility. Also, a need exists to provide a closure tape tab which can be made in a cost-efficient way.

The present disclosure relates to a closure tape tab for fastening of an absorbent article on the body of a person. The closure tape tab comprises a proximal end portion and a distal end portion being connected by an inner tab portion. The inner tab portion is formed from a backing comprising mechanical fastening elements protruding from one of the first or the second major surface thereof. The first and second major surfaces of the inner tab portion are understood to be opposite to each other.

Typically, a closure tape tab has a proximal and a distal end. The proximal end is oriented towards the diaper edge, whereas the distal end faces away from the diaper edge. Such a closure tape tab is typically anchored to the diaper to one of its end portions (proximal end portion) by means of, for example, a pressure sensitive adhesive layer. The proximal end portion typically extends from the proximal end towards the distal end and the distal end portion typically extends from the distal end to the proximal end.

A portion of the closure tape tab is understood as a discrete or individual part, e. g. separate from another portion of the closure tape tab. Typically, the portions have a backing which is a discrete part of a thin, elongate material such as a film, a fibrous material or a fabric. For example, the backing has a thickness of 10 to 1000 µm. Typically, the backing has a basis weight of 100 g/m², preferably 70 g/m², more preferred 60 g/m², most preferred 40 g/m². In certain embodiments, the backing may even have a lower basis weight than 40 g/m². Suitable materials for the backing include polypropylene, polyethylene, polyethylene terephthalate, polyvinyl chloride or combinations thereof. Typically, a closure tape tab has an elongated shape of 30 to 150 mm in length (i. e. main extension in elongation), 10 to 50 mm in width (i. e. perpendicular to the length). The longitudinal extension of the closure tape tab is the extension parallel to main extension of the closure tape tab, which corresponds to the direction perpendicular to the machine direction (MD). This direction is also referred to as cross direction (CD).

A closure tape tab according to the present disclosure is advantageous because the tab is formed from two portions connected by the inner portion which increases the flexibility of the tab, in particular in the region of the inner tab portion comprising the mechanical fastening elements. Also, such a closure tape tab provides the possibility to provide an absorbent article that has an improved conformability and thus prevents unwanted pop-open of the closure tape tab in case of movement of a wearer of the absorbent article to which the closure tape tab may be attached.

The present disclosure furthermore relates to a closure tape comprising a plurality of closure tape tabs according to the present disclosure in an endless form. Individual closure tape tabs can be cut from the tape. Optionally, the tape is wound up into a roll. Such a closure tape is advantageous because it allows for an easy and reliable way to manufacture closure tape tabs therefrom. A roll of tape is beneficial because it represents an easy, reliable and space-saving way of storing such a closure tape prior to manufacture of closure tape tabs and of handling it during manufacture. It can easily be unwound to provide the closure tape such that closure tape tabs can be cut therefrom easily and efficiently. The longitudinal extension of the closure tape corresponds to the machine direction (MD). The direction perpendicular to that direction is referred to as the cross direction (CD).

The present disclosure moreover relates to a method of making a closure tape from which closure tape tabs according to the present disclosure can be cut. The method comprises the steps of
a)providing a first tape portion forming a proximal end portion;
b)providing a second tape portion forming a distal end portion;
c)connecting said first and second tape portions by means of an inner tab portion having a first major surface and an opposite second major surface. Said proximal and distal end portions are connected to one of the major surfaces thereof. The proximal and the distal end portions form discrete portions, preferably such that opposing ends of said proximal and distal end portions are spaced from each other. The inner tab portion is formed from a backing comprising mechanical fastening elements, protruding from one of the first or the second major surface thereof.

The method includes optionally winding the closure tape up the tape into a roll. Such a method is advantageous because it is an easy and reliable way to manufacture closure tape. A roll of tape is beneficial because it represents an easy, reliable and space-saving way of storing such a closure tape prior to manufacture of closure tape tabs and of handling it during manufacture. It can easily be unwound to provide the closure tape such that closure tape tabs can be cut therefrom easily and efficiently.

The present disclosure also relates to a method of making closure tape tabs from a closure tape. The method comprises the step of providing a closure tape according to the present disclosure, optionally the step of unwinding the tape from the roll. The method additionally comprises the step of applying cuts in cross direction to form individual tape tabs. Such a method is advantageous because it is an easy and reliable way to manufacture closure tape. A roll of tape is beneficial because it represents an easy, reliable and space-saving way of storing such a closure tape prior to manufacture of closure tape tabs and of handling it during manufacture. It can easily be unwound to provide the closure tape such that closure tape tabs can be cut therefrom easily and efficiently.

The present disclosure relates to an absorbent article such as a disposable diaper comprising at least one closure tape tab according to the present disclosure. Such absorbent articles may include disposable diaper or sanitary napkins. Absorbent articles having such a closure tape tabs are advantageous because these are easy to use, i. e. to fasten and unfasten. Also, such closure tape tabs provide a sufficient wearing comfort of an absorbent article to which the tabs are attached. The flexibility and conformability of the closure tape tabs help to increase the wearing comfort and reduce the risk of premature or unwanted pop-open of the closure tape tabs and thus unfastening of the article, respectively in case of movement of a wearer of the absorbent article to which the closure tape tab may be attached.

In one embodiment, the proximal and the distal end portions of the closure tape tab form discrete portions. Such a closure tape tab is advantageous because - as mentioned above - the flexibility and conformability of the tab is increased and the risk for unwanted pop-open of the closure tape tab is reduced.

In another embodiment, the proximal and the distal end portion of the closure tape tab have opposing edges facing to each other. The opposing edges overlap with the inner tab portion such that the edges are arranged on one of the first or second major surface of the inner tab portion. Such a closure tape tab is advantageous because the overlap between the inner tab portion and the proximal and distal end portions allows for an easy and reliable connection of the portions.

In yet another embodiment, the first and/or second major surface of the inner tab portion of the closure tape tab is not entirely covered by the proximal and the distal end portions such that the proximal and the distal end portions are spaced from each other. Such a spaced configuration of the proximal and distal end portions even further enhances the flexibility of the closure tape tab and thereby further increases the flexibility and conformability and reduces the risk of unwanted pop-open of the closure tape tab. Also, such a spaced configuration helps to save material costs, i. e. there is less material needed underneath of the inner tab portion.

In a further embodiment, the fastening elements of the closure tape tab are male fastening elements comprising a stem projecting from a major surface of the inner tab portion. Such a closure tape tab with male fastening elements increases the engageability of the fasteners on the inner tab portion to a corresponding female fastening material such as a fibrous or fabric material, for example a non-woven, a loop material etc.

In certain further embodiments, the stems of the male fastening elements of the closure tape tab comprise an enlarged section which is positioned at the end of the stem opposite of the surface of the backing. Such a closure tape tab with male fastening elements increases the engageability of the fasteners on the inner tab portion to a corresponding female fastening material such as a fibrous or fabric material, for example a non-woven, a loop material etc. Using male fastening elements with enlarged sections even further increase the engageability of the fasteners to a female fastening material.

In certain further embodiments, the enlarged portions of the male fastening elements of the closure tape tab form hooks, T's, J's, mushroom-type heads. Such a closure tape tab with male fastening elements increases the engageability of the fasteners on the inner tab portion to a corresponding female fastening material such as a fibrous or fabric material, for example a non-woven, a loop material etc. Using the above-mentioned specific shapes of male fastening elements even further increase the engageability of the fasteners to a female fastening material.

Male fastening elements, including hook shapes, suitable for the closure tape tab are, for example, disclosed in US 5,868,987 (3M, Kampfer); US 5,900,360 (Velcro, Provost); US 6,054,091 (3M; Miller) or WO 13/40156 (3M, Pariseau et al.).

In another embodiment, the proximal and distal end portions of the closure tape tab are connected to the inner tab portion by means selected from adhesive, heat-bonding such as ultrasonic bonding, mechanical bonding and combinations thereof. Suitable adhesives for connecting the portions of the closure tape tab include pressure-sensitive adhesives or hotmelt adhesives. In case an adhesive is used, typically an adhesive layer of about 30 g/m² is applied to one of the major surfaces of the closure tape tab. Suitable adhesives are disclosed in EP 996 402 (3M, Guenther et al.). Using such means for connecting the portions of the closure tape tab provides for a reliable and cost-efficient way of connection while providing the required bonding strength.

In yet another embodiment, the mechanical fastening elements protrude from the second major surface of the inner tab portion. The advantage of such an arrangement is that the other of the major surfaces, i. e. the first major surface is available for bonding to the proximal and distal portions without inference by mechanical fastening elements.

In a further embodiment, the second major surfaces of the proximal and distal end portions each comprise an adhesive layer. The first and second major surfaces of the proximal end portion are understood to be opposite to each other. The first and second major surfaces of the distal end portion are understood to be opposite to each other. The advantage of such adhesive layers is that these can also be used to attach the closure tape tab to an absorbent article with these adhesives. Thus, no extra attachment means such as an adhesive is necessary for attaching the closure tape tab to the absorbent article.

In another embodiment, the proximal and distal end portions of the closure tape tab are connected to the first major surface of the inner tab portion. Such an arrangement is beneficial as the other major surface, i. e. the second major surface, is available for arranging mechanical fastening elements thereon without any interference of a connection between the proximal and distal end portions to the inner tab portion.

In certain embodiments, the second major surfaces of the proximal and distal end portions of the closure tape tab are connected to the first major surface of the inner tab portion through the adhesive layers. As mentioned above, such adhesive layers are advantageous as these may be used to attach the closure tape tab to an absorbent article so that no extra adhesive layer is necessary for attachment of the closure tape tab to an absorbent article.

In one embodiment, each of the proximal and the distal end portions of the closure tape tab cover 50 % or less, preferably 40 % or less, more preferably 30 % or less and most preferably 20 % or less of the first major surface of the inner tab portion. The minimum coverage of the inner tab portion by each of the proximal and distal end portion is 15 % or 3 mm. Also, such a spaced configuration, i. e. where the proximal and the distal end portions each cover less than 50 % of the first major surface of the inner tab portion, does not only increase the flexibility of the closure tape tab as mentioned above, but also helps to save material costs, i. e. there is less material needed underneath of the inner tab portion.

In another embodiment, the coverage of the inner tab portion by the proximal and distal end portion may be asymmetric. For example, the proximal portion of the closure tape tab may cover 70 % or less and the distal end portion may cover 30 % or less of the inner tab portion. Preferably, the proximal portion of the closure tape tab may cover 60 % or less and the distal end portion may cover 40 % or less of the inner tab portion. The minimum coverage of the inner tab portion by each of the proximal and distal end portion is 15 % or 3 mm. Such an asymmetric arrangement is beneficial as the coverage between proximal end portion and inner tab portion is higher and thus stronger, so that it can carry higher load, e. g. introduced by the force to fasten the absorbent article to which the closure tape tab can be attached. Alternatively, the proximal portion of the closure tape tab may cover 40 % or less and the distal end portion may cover 60 % or less of the inner tab portion. Preferably, the proximal portion of the closure tape tab may cover 30 % or less and the distal end portion may cover 70 % or less of the inner tab portion.

In yet a further embodiment, the longitudinal extension of the distal end portion of the closure tape tab is smaller than the longitudinal extension of the proximal end portion. The longitudinal extension of the proximal and of distal end portion is the extension parallel to main extension of the closure tape tab, which corresponds to the direction perpendicular to the machine direction (MD). This direction is also referred to as cross direction (CD). Such a closure tape tab is advantageous as it provides for a good haptic for a user of the closure tape tab when gripping to for fastening or unfastening an absorbent article to which such a closure tape tab has been attached. Also, a proximal end portion larger in extension than the distal end portion provides for more flexibility when fastening an absorbent article to which the closure tape tab may be attached because the distance at which the inner tab portion with the mechanical fastening elements is arranged from the edge of the article is increased.

In another embodiment, the thickness of the distal end portion of the closure tape tab is different from the thickness of the proximal end portion, in particular less than the thickness of the proximal end portion. For example, the thickness of the distal end portion may be less than 70 %, preferably less than 50 %, more preferred less than 30 % of the thickness of the proximal end portion. The thickness of the proximal end portion may, for example, be 100 µm and the thickness of the distal end portion may be 70 µm, preferably 50 µm, more preferred 30 µm. Such an arrangement with different thicknesses of the proximal and distal end portion of the closure tape tab is advantageous because it helps saving material with a thinner distal end portion while the proximal end portion is still strong enough to carry the force applied to the closure tape when fastened. Also, a thinner distal end portion provides for more flexibility of the closure tape tab in the region of the distal end portion.

In another embodiment, the basis weight of the distal end portion of the closure tape tab is different from the basis weight of the proximal end portion, in particular less than the thickness of the proximal end portion. For example, the basis weight of the distal end portion may be less than 70 %, preferably less than 50 %, more preferred less than 30 % of the basis weight of the proximal end portion. The basis weight of the proximal end portion may, for example, be 100 g/m² and the basis weight of the distal end portion may be 70 g/m², preferably 50 g/m², more preferred 30 g/m². Other configurations are conceivable. Such an arrangement with different basis weights of the proximal and distal end portion of the closure tape tab is advantageous because it helps saving material with a lighter distal end portion while the proximal end portion is still strong enough to carry the force applied to the closure tape when fastened. Also, a lighter distal end portion provides for more flexibility of the closure tape tab in the region of the distal end portion.

In yet another embodiment, the proximal and/or the distal end portion of the closure tape tab comprises a non-woven material. For example, non-woven materials include carded non-wovens, spunbond non-wovens, melt-blown non-wovens or combinations thereof. Suitable non-woven materials are disclosed in EP 1 133 967 (3M, Kinnear et al.), EP 1 599 160 (3M, Loescher). Such a closure tape tab comprising a non-woven material is beneficial as it has a soft surface providing for a good haptic for the user as well as for a cloth-like feel of the surface. Alternatively, a loop material may be used such as a so-called Extrusion Bonded Loop (EBL) as, for example, described in US 5,888,607 (3M, Seth et al.) or so-called Knitted Loop as, for example, described in US 2008/009819 (3M, Liu et al.).

In a further embodiment, the inner tab portion of the closure tape tab comprises an elastic material. Such a closure tape tab may even further increase comfort of wearing and flexibility of fastening an absorbent article to which the closure tape tab may be attached, in particular when a person wearing the article is moving. Also, the risk of a leakage is minimized as the article is still fixedly secured to the wearer even when moving.

In yet a further embodiment, the closure tape tab comprises two proximal portions connected by an elastic portion. Such a closure tape tab may even further increase comfort of wearing and flexibility of fastening an absorbent article to which the closure tape tab may be attached, in particular when a person wearing the article is moving. Also, the risk of a leakage is minimized as the article is still fixedly secured to the wearer even when moving. Also, separating the proximal end portion into a first and second proximal end portion even further helps to increase flexibility and to reduce rigidity in this region. In certain embodiments, the first and second proximal end portions are arranged such that a gap is provided between these. Alternative, in certain other embodiments, the first and second proximal portions are arranged close to each other or even touching each other, so that no gap is provided.

Elastic materials suitable for the closure tape tab are, for example, disclosed in EP 0 996 402 B1 (3M, Guenther et al.); EP 1 599 160 B1 (3M, Loescher), EP 1 133 967 A1 (3M, Kinnear et al.), EP 500 590 (3M, Hanschen et al.), EP 2 882 592 (3M, Hanschen et al.), EP 1 900 512 (3M, Jaeger et al.) or EP 2 340 005 (3M, Jaeger et al.).

In one embodiment, a finger lift is attached to an adhesive layer present on the first or second major surface of the distal end portion to cover the adhesive. Such finger lifts include a film or non-woven material. Typically, the finger lift has the same extension in machine direction (i. e. the direction parallel to the longitudinal extension of the closure tape tab) and preferably between 8 and 30 mm in cross direction (i. e. the direction perpendicular to the main extension of the closure tape tab). The finger lift increases the haptic for the user and helps the user to grip the distal end portion of the closure tape tab for fastening or unfastening of an absorbent article to which the closure tape tab may be attached.

In one embodiment, the closure tape tab comprises an anti-adhesive coating on one of the first or second major surface of the proximal and distal end portions. The anti-adhesive coating may comprise a low-adhesive backsize (LAB) coating. Typically, a silicon coating is used as LAB coating. It is advantageous to provide such an anti-adhesive coating on the closure tape tab as it prevents adhesive otherwise sticking to a major surface covered with that coating. Blocking of a closure tape when unwinding such a tape from a roll to cut such closure tape tabs from the closure tape is thus prevented. Suitable anti-adhesive coatings are, for example, described in EP 1 697 134 B1 (3M, Selen et al.). Alternative, a liner may be used to cover a surface which may come into contact with an adhesive in order to prevent blocking during manufacture of closure tape tabs from a closure tape. Typically, such liners comprise a paper or film backing with a siliconized surface. The use of such a liner may be advantageous because it avoids the need for an anti-adhesive coating or treatment of the major surfaces of the proximal and/or distal end portions while still preventing blocking of the closure tape when unwinding it.

The invention was described in various embodiments above. It is understood by a person skilled in the art, that one, several or all of the above-mentioned embodiments can be combined with each other.

The invention will now be described in more detail with reference to the following Figures exemplifying particular embodiments of the invention:
- Fig. 1: is a schematic side view of a closure tape tab according to the present disclosure.
- Fig. 2: is a schematic side view of a closure tape tab according to another embodiment of the present disclosure.
- Fig. 3: is a schematic side view of a closure tape tab according to a further embodiment of the present disclosure.
- Fig. 4: is a schematic side view of a closure tape tab according to yet a further embodiment of the present disclosure.
- Fig. 5: is a schematic side view of a closure tape tab according to yet another embodiment of the present disclosure.
- Fig. 6: is a schematic side view of a closure tape tab according to a further embodiment of the present disclosure.
- Fig. 7: is a perspective view of a roll of closure tape and a roll according to the present disclosure as well as of a side view of a closure tape tab according to the present disclosure cut from the tape.
- Fig. 8: is a perspective view of an absorbent article such as a disposable diaper comprising a closure tape tab according to the present disclosure attached to each of the longitudinal edges of the article.

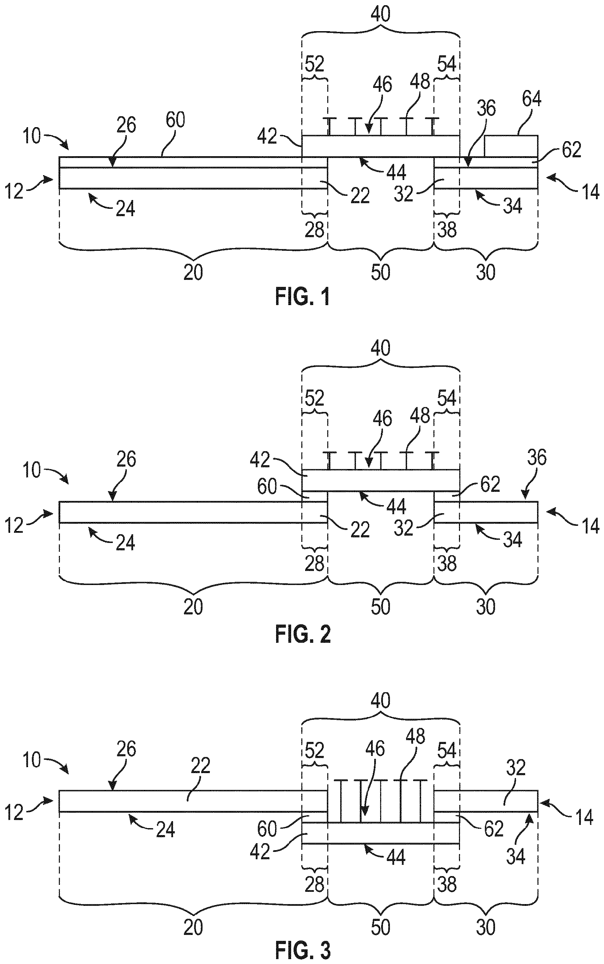

Fig. 1 illustrates an embodiment of closure tape tab 10 according to the present disclosure in a side view. The closure tape tab 10 comprises a proximal end 12 and a proximal end portion 20 extending therefrom. The proximal end portion 20 further comprises a backing 22 and a first and second major surface 24, 26. In the embodiment shown in Fig. 1, an adhesive layer 60 is arranged on the second major side 26. The closure tape tab 10 further comprises - opposite to the proximal end 12 - a distal end 14 and a distal end portion 30 extending therefrom towards the proximal end. The distal end portion 30 further comprises a backing 32 and a first and second major surface 34, 36. In the embodiment shown in Fig. 1, an adhesive layer 62 is arranged on the second major side 36.

The proximal and the distal end portions 20, 30 are connected by an inner tab portion 40 formed from a backing 42, comprising a first and second major surface 44, 46. In the embodiment shown, the inner tab portion 40 is connected to the proximal and distal end portion 20, 30 by adhesive layers 60, 62, whereby the attachment portions 52, 54 of the inner tab portion 40 contact with its first major side 44 the adhesive layers 60, 62 in an attachment region 28, 38 of the proximal and distal end portions 20, 30. The proximal end portion 20 constitutes the attachment portion or manufacturer's end of the closure tape tab 10 with which the closure tape tab is attached to an absorbent article such as a disposable diaper. The proximal and distal end portions 20, 30 do not cover the first major surface 44 of the inner tab portion 40 entirely such that the proximal and distal end portions 20, 30 are spaced from each other. In other words, the areal extension of the attachment regions 52, 54 is smaller than that of the entire major surface 44 of the inner tab portion 40 and thus, a gap 50 is provided between the proximal and distal end portions 20, 30. The inner tab portion 40 further comprises mechanical fastening elements 46 protruding from the second major surface 46 of the inner tab portion 40.

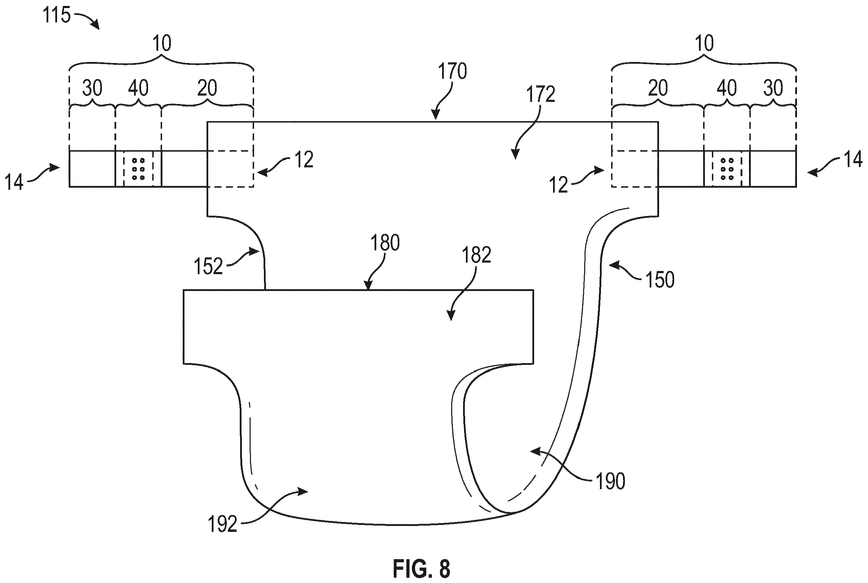

In the embodiment shown, the mechanical fastening elements 48 are male fastening elements 48 having a stem and an enlarged section positioned at the end of the stem opposite to the second major surface 46. Consequently, the adhesives 60, 62 and the mechanical fastening elements 48 are on the opposite major surfaces 44, 46 of the inner tab portion 40. Thereby, the inner tab portion 40 constitutes the fastening portion of the closure tape tab 10. The closure tape tab 10 further comprises a finger lift 64, which is arranged on the adhesive layer 62 of the distal end portion 30 at the region opposite to the attachment region 54 of the inner tab portion 40. The finger lift 64 covers the adhesive layer 62 and facilitates easier gripping of the closure tape tab 10 by a user of the absorbent article 115 (not shown in Fig. 1, see Fig. 8) to which the closure tape tab 10 may be attached. The distal end portion 30 constitutes the user's end portion of the closure tape tab 10 at which a user of the absorbent article, to which the closure tape tab 10 may be attached, can grip the closure tape tab 10 for fastening or unfastening the article.

Fig. 2 illustrates another embodiment of the closure tape tab 10 according to the present disclosure. The closure tape tab 10 is similar to the closure tape tab 10 as shown in Fig. 1 except that the extension of the adhesive layer 60, 62 is different. The adhesive layers 60, 62 are only provided on the second major sides 26, 36 of the proximal and distal end portions 20, 30 in the attachment regions 28, 38 thereof. Thus, most of the second major surfaces 26, 36 is free of an adhesive in this embodiment. There is no finger lift necessary in such a configuration because the distal end 14 can easily be gripped by a user of the absorbent article 115 (not shown in Fig. 1, see Fig. 8) to which the closure tape tab 10 may be attached as there is no adhesive in this region.

Fig. 3 shows a further embodiment of the closure tape tab 10 according to the present disclosure. The closure tape tab 10 is similar to the embodiment as shown in Fig. 2 except that the adhesive layers 60, 62 are provided on the first major surfaces 24, 34 of the proximal and distal end portions 20, 30 of the closure tape tab 10 contacting the second major surface 46 of the inner tab portion 40 in the attachment regions 52, 54 thereof. Similar to Fig. 2, the adhesive layers 60, 62 are only provided in the attachment regions 28, 38 of the proximal and distal end portions 20, 30 of the closure tape tab 10, but different to Fig. 2 on the first major surfaces 24, 34. Similar to Figs. 1 and 2, the inner tab portion 40 is formed from a backing 42 comprising mechanical fastening elements 48 protruding from the second major surface 46 of the inner tab portion 40. Consequently, the adhesives 60, 62 and the mechanical fastening elements 48 are on the same major surface, i. e. the second major surface 46, of the inner tab portion 40. In the embodiment shown, the mechanical fastening elements 48 are male fastening elements 48 having a stem and an enlarged section positioned at the end of the stem opposite to the major surface 46. Most of the first major surfaces 24, 34 is free of an adhesive in this embodiment and no finger lift is necessary in this embodiment. The second major surfaces 26, 36 are entirely free of adhesive in this embodiment.

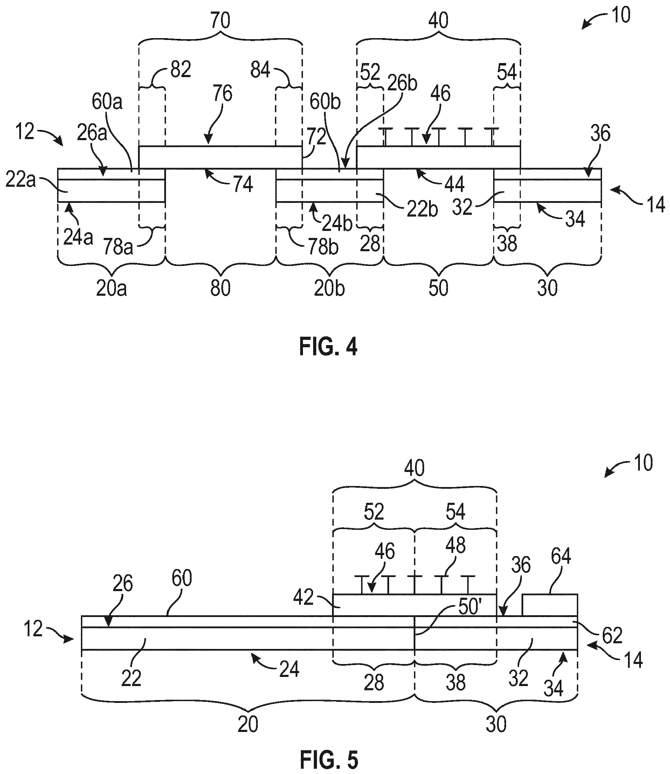

Fig. 4 illustrates a further embodiment of the closure tape tab 10 according to the present disclosure. Here, the proximal end portion 20 was replaced by a first and second proximal end portion 20a, 20b connected by an elastic portion 70. The first and second proximal end portion comprises a first and second backing 22a, 22b which replaces the backing 22 of the proximal end portion 20 in the embodiments shown in Fig. 1 to 3. The first and second proximal end portions 20a, 20b comprise a first and second major surface 24a, 24b, 26a, 26b, wherein the adhesive layers 60a, 60b are provided on the second major surfaces 26a, 26b of the first and second proximal end portion 20a, 20b. Similar to the connection between second proximal and the distal end portions 20b, 30 of the closure tape tab 10, the connection between the elastic portion 70 and the first and second proximal end portions 20a, 20b is achieved by the adhesive layers 60a, 60b on the second major side 26a, 26b of the first and second proximal end portions 20a, 20b in the attachment regions 78a, 78b being in contact with the attachment regions 82, 84 of the first major surface 74 of the elastic portion 70. Although adhesive layers 60a, 60b extend onto the entire second major surfaces 26a, 26b of the first and second proximal portions 20a, 20b, it is also conceivable that the adhesive layers 60a, 60b only extends in the attachment regions 78a, 78b of the second major surface 26a, 26b of the first and second proximal end portions 20a, 20b. In the embodiment shown in Fig. 4, the first and second proximal end portions 20a, 20b do not cover the first major surface 74 of the elastic portion 70 such that the first and second proximal end portions 20a, 20b are spaced from each other. In other words, the areal extension of the attachment regions 78a, 78b is smaller than that of the entire major surface 74 of the elastic portion 70 and thus, a gap 80 is provided between the first and second proximal end portions 20a, 20b. It is also conceivable that the first and second proximal portions are such arranged that no gap is provided (not shown). The closure tape tab 10 may further comprise a finger lift 64, which is omitted in Fig. 4.

Fig. 5 shows a further embodiment of the closure tape tab 10 according to the present disclosure. The closure tape tab 10 is similar to the closure tape tab 10 as shown in Fig. 1 except that the proximal and distal end portions 20, 30 are connected by the inner tab portion 40 in a different way. As shown, the attachment regions 28, 38 of the proximal and distal end portions 20, 30 cover the first major surface 44 of the inner tab portion 40 entirely such that the proximal and distal end portions 20, 30 are not spaced from each other. Thus, no gap is provided between the proximal and distal end portions 20, 30 as shown in Fig. 1. Instead, the embodiment of Fig. 5 shows that the proximal and distal end portions 20, 30 are close to each other or even touch each other as indicated by 50'. Consequently, the attachment portions 28, 38 of the proximal and distal end portions 20, 30 and the respective attachment regions 52, 54 on the inner tab portion 40 are larger in area compared to Fig. 1.

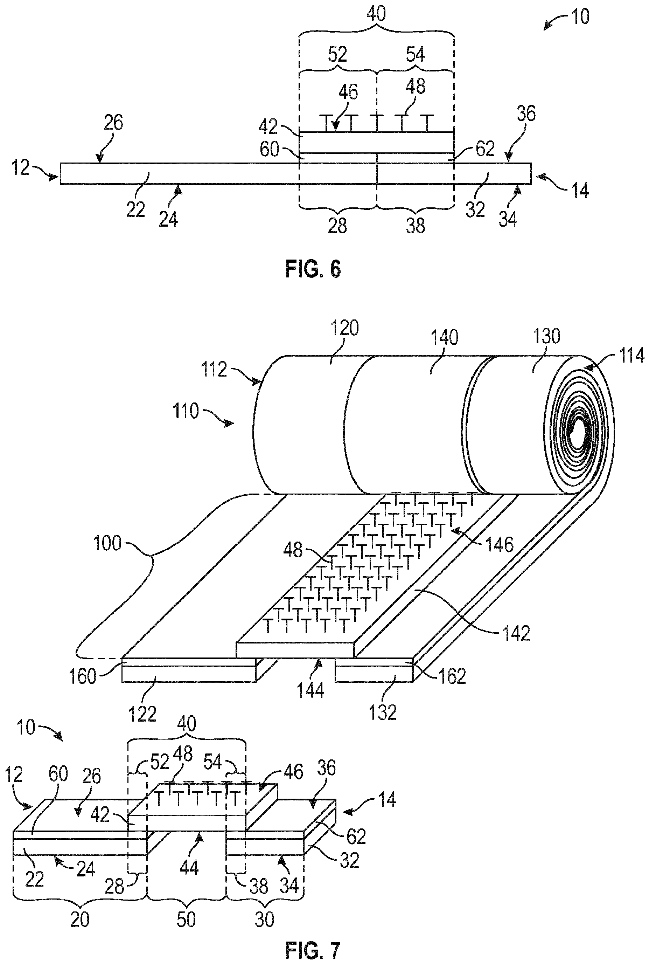

Fig. 6 shows a further embodiment of the closure tape tab 10 according to the present disclosure. The closure tape tab 10 is similar to the closure tape tab 10 as shown in Fig. 2 except that the proximal and distal end portions 20, 30 are connected by the inner tab portion 40 in a different way. As shown, the attachment regions 28, 38 of the proximal and distal end portions 20, 30 cover the first major surface 44 of the inner tab portion 40 entirely such that the proximal and distal end portions 20, 30 are not spaced from each other. Thus, no gap is provided between the proximal and distal end portions 20, 30 as shown in Fig. 2. Instead, the embodiment of Fig. 5 shows that the proximal and distal end portions 20, 30 are close to each other or even touch each other as indicated by 50'. Consequently, the attachment portions 28, 38 of the proximal and distal end portions 20, 30 and the respective attachment regions 52, 54 on the inner tab portion 40 are larger in area compared to Fig. 1. The embodiment shown in Fig. 6 has some similarity to that shown in Fig. 5, except that the extension of the adhesive layers 60, 62 is similar to the embodiment as shown in Fig. 2. That is, the adhesive layers 60, 62 are only provided on the second major sides 26, 36 of the proximal and distal end portions 20, 30 in the attachment regions 28, 38 thereof. Thus, most of the second major surfaces 26, 36 is free of an adhesive in this embodiment. There is no finger lift necessary in such a configuration because the distal end 14 can easily be gripped by a user of the absorbent article 115 (not shown in Fig. 1, see Fig. 8) to which the closure tape tab 10 may be attached as there is no adhesive in this region.

Fig. 7 illustrates a roll 110 of closure tape 100 having a proximal end 112 and a distal end 114. The roll 110 and the closure tape 100, respectively, comprise a proximal end portion web 120 and a distal end portion web 130 being connected by an inner tab portion web 140. The proximal end portion web 120 extends from the proximal end 112 towards the distal end 114 and the distal end portion web 130 extends from the distal end 114 towards the proximal end 112. The inner tab portion web 140 is arranged between the proximal end portion web 120 and the distal end portion web 130 and connects the proximal and distal end portion webs 120, 130 by means of adhesive layers 160, 162. Adhesive layers 160, 162 are arranged on the second major sides of the proximal and distal portion webs 120, 130 and are in contact with the first major surface 144 of the inner tab portion web 140. Fig. 7 further shows several layers of closure tape 100 wound up into the roll 110 and a length of closure tape 100 unwound from the roll 110. The inner tab portion web 140 is formed from a backing 142 and further comprises mechanical fastening elements 46 protruding from the second major surface 146 of the inner tab portion web 140. In the embodiment shown, the mechanical fastening elements 48 are male fastening elements 48 having a stem and an enlarged section positioned at the end of the stem opposite to the major surface 146. Consequently, the adhesives 160, 162 contacting the first major surface 144 of the inner tab portion web 140 and the mechanical fastening elements 48 protruding from the second major surface 146 are on the different major surfaces 144, 146 of the inner tab portion web 140. The closure tape 100 may further comprise a web of finger lifts, which is omitted in Fig. 7. Fig. 7 further shows in a perspective view a closure tape tab 10 cut from the closure tape 100 in cross direction (CD), i. e. transverse to the main extension direction or machine direction (MD) of the closure tape 100. The closure tape tab 10 is identical to the one as shown in Fig. 1, except that the finger lift 64, which is shown in Fig. 1, was omitted in Fig. 7.

Fig. 8 is a perspective view of an absorbent article 115 such as a disposable diaper 115. The diaper 115 comprises an inner surface forming a topsheet 190, an outer surface forming a backsheet 192 and an absorbent core in between (not shown here). Typically, the outer surface or backsheet 192 of the diaper is impervious to liquids and is preferably made of a flexible thin plastic film or a fibrous material such as a non-woven which is rendered liquid impervious by suitable treatment. The outer surface or backsheet 192 preferably has a cloth-like feel. A suitable backsheet 192 may be, for example, a spunbond polypropylene non-woven of approximately 14 - 35 g/m² basis weight. The inner surface or topsheet 190, respectively, is liquid pervious and preferably made of from a wide range of materials such as porous foams, reticulated foams, aperture films, natural fibers (e. g. wood or cotton fibers), synthetic fibers (e. g. polyester, polypropylene or polyethylene fibers) or from combinations of natural and synthetic fibers. Preferably, the topsheet 190 is made from a hydrophobic material to isolate the wearer's skin from liquids retained in the absorbent core. A suitable topsheet may, for example, be a spun-bond or carded polypropylene non-woven of approximately 15 - 25 g/m² basis weight. The topsheet 190 and the backsheet 192 may be joined to each other directly or indirectly by various means comprising, for example, pressure-sensitive adhesives, hot-melt adhesives or other adhesives, ultrasonic bonding and/or heat and/or pressure.

The diaper 115 further comprises a rear and front transverse edge 170, 180, from which the rear and front waist regions 172, 182 extend. The diaper 115 further comprises longitudinal side edges 150, 152 extending from the rear transverse edge 170 to the front transverse edge 180. A closure tape tab 10 according to the present disclosure is arranged at each longitudinal side 150, 152 at the rear waist region 172. The closure tape tabs 10 comprise a proximal end portion 20 and a distal end portion 30 being connected by an inner tab portion 40 which is formed from a backing 42 (not indicated here) comprising mechanical fastening elements 48 protruding the second major surface 46 of the inner tab portion 40. The closure tape tabs 10 are attached to the outer surface 192 of the diaper 115 in the rear waist region 172 at the longitudinal side edges 150, 152 of the diaper 115 with their proximal end portions 20 as indicated by a dotted line. The distal end 12 and the distal end portion 30 extend outward from the longitudinal edges 150, 152 of the disposable diaper 115. In use, the inner tab portion 40 of the closure tape tab 10 will be brought into contact with the backsheet 192 in the front wait region 182 such that the male fastening elements 48 of the inner tab portion 40 engage with the fibrous material or fabric (not shown) of the backsheet 192.

## Claims

1. A closure tape tab (10) for fastening of an absorbent article (115) on the body of a person, comprising:
- a proximal end portion (20) and a distal end portion (30) being connected by an inner tab portion (40), wherein the inner tab portion (40) is formed from a backing (42) comprising mechanical fastening elements (48) protruding from one of the first or the second major surface (44, 46) thereof.

2. The closure tape tab (10) according to claim 1, wherein the proximal and the distal end portions (20, 30) form discrete portions (20, 30).

3. The closure tape tab (10) according to claim 1 or 2, wherein the first and/or second major surface (44, 46) of the inner tab portion (40) is not entirely covered by the proximal and the distal end portions (20, 30) such that the proximal and the distal end portions (20, 30) are spaced from each other.

4. The closure tape tab (10) according to any one of the preceding claims, wherein the mechanical fastening elements (48) are male fastening elements (48) comprising a stem protruding from one of the first or second major surface (44, 46) of the inner tab portion (40).

5. The closure tape tab (10) according to claim 4, wherein the stems comprise an enlarged section which is positioned at the end of the stem opposite of the surface of the backing (42).

6. The closure tape tab (10) according to any one of the preceding claims, wherein the mechanical fastening elements (48) protrude from the second major surface (46) of the inner tab portion (40).

7. The closure tape tab (10) according to any one of the preceding claims, wherein said proximal and distal end portions (20, 30) are connected to the first major surface (44) of the inner tab portion (40).

8. The closure tape tab (10) according to any one of the preceding claims, wherein the second major surfaces (26, 36) of the proximal and distal end portions (20, 30) each comprise an adhesive layer (60, 62).

9. The closure tape tab (10) according to claim 8, wherein the second major surfaces (26, 36) of the proximal and distal end portions (20, 30) are connected to the first major surface of the inner tab portion (40) through the adhesive layers (60, 62).

10. The closure tape tab (10) according to any one of the preceding claims, wherein the longitudinal extension of the distal end portion (30) is smaller than the longitudinal extension of the proximal end portion (20).

11. The closure tape tab (10) according to any one of the preceding claims, wherein the closure tape tab (10) comprises two proximal portions (20a, 20b) connected by an elastic portion (80).

12. A closure tape (100) comprising a plurality of closure tape tabs (10) according to any one of the preceding claims in an endless form from which individual closure tape tabs (10) can be cut, optionally wound up into a roll (110).

13. A method of making a closure tape (100) from which closure tape tabs (10) according to any one of claims 1 to 11 can be cut, the method comprising the steps of:
a. providing a first tape portion forming a proximal end portion (20);
b. providing a second tape portion forming a distal end portion (30);
c. connecting said first and second tape portions by means of an inner tab portion (40) having a first major surface (44) and an opposite second major surface (46), wherein said proximal and distal end portions (20, 30) are connected to one of the major surfaces (44, 46) thereof, wherein the proximal and the distal end portions form discrete portions, preferably such that opposing ends (22, 32) of said proximal and distal end portions (20, 30) are spaced from each other;
wherein the inner tab portion (40) is formed from a backing (42) comprising mechanical fastening elements (48), protruding from one of the first or the second major surface (44, 46) thereof, and
d. optionally winding up the tape into a roll.

14. The method of making closure tape tabs (10) from a closure tape (100), the method comprising the step of providing a closure tape (100) according to claim 12, optionally the step of unwinding the tape (100) from the roll (110), the method additionally comprising the step of applying cuts in cross direction to form individual tape tabs (10).

15. An absorbent article (115) such as a disposable diaper (115) comprising at least one closure tape tab (10) according to any one of claims 1 to 11.
